# EUROPÄISCHE PATENTSCHRIFT

(11) **EP 2 346 533 B1**
(45) Veröffentlichungstag und Bekanntmachung des Hinweises auf die Patenterteilung: **12.03.2014**
(21) Anmeldenummer: 08875158.1
(22) Anmeldetag: 07.10.2008
(51) Int. Cl.: A61L 2/07, A61L 2/26, B08B 3/04

(54) **VORRICHTUNG ZUR BEHANDLUNG KLEINER TEILE**
DEVICE FOR TREATING SMALL PARTS
DISPOSITIF POUR TRAITER DE PETITES PIÈCES

(43) Veröffentlichungstag der Anmeldung: 27.07.2011
(73) Patentinhaber: Netzhammer, Eric, 4103 Bottmingen (CH)
(72) Erfinder: Netzhammer, Eric, 4103 Bottmingen (CH)
(74) Vertreter: Braun, André jr.
(86) Internationale Anmeldenummer: PCT/EP2008/063390
(87) Internationale Veröffentlichungsnummer: WO 2010/040383

(56) Entgegenhaltungen:
- WO-A-03/009942
- WO-A-2004/029351
- DE-A1- 10 235 374
- US-A- 2 795 404

## Beschreibung

Die Erfindung betrifft eine Vorrichtung zum schonenden Reinigen, Sterilisieren und Trocknen grosser Mengen von kleinen Teilen. Diese Teile können zum Beispiel Teile von Spritzen oder von Ampullen sein, also Teile welche vorwiegend im medizinischen Bereich eingesetzt werden.

Für die Durchführung dieser Prozesse sind verschiedene Maschinen und Verfahren bekannt, die aber mit Nachteilen behaftet sind, sei es im Bereich der Verfahren oder im Bereich der Ausführung der Maschine.

Eine bekannte Einrichtung besteht aus einem mobilen Behandlungsbehälter mit einem rotierenden Innenkorb, in welchen die Teile gegeben werden. Der Korb ist grob perforiert, ähnlich einer Kleiderwaschmaschine. Nach erfolgter Behandlung werden die Teile in diesem Behandlungsbehälter zum Verbraucher transportiert und an diesen übergeben. Nachteilig bei dieser Einrichtung ist, dass die grobe Perforation ein schlechtes Wirbelbett erzeugt und deshalb keine gleichmässige Behandlung gegeben ist, indem der Medienstrom unkontrolliert durch die zu behandelnden Teile strömt. Besonders nachteilig ist, dass der Medienstrom sogar ausserhalb des Behandlungskorbes vorbei strömen kann und dadurch die Teile überhaupt nicht mit den Medien in Berührung kommen und somit nicht behandelt werden.

Eine weitere bekannte Einrichtung besteht aus einem rotierenden mobilen Behandlungsbehälter mit einer Siebplatte bzw. einem feinmaschigen Sieb, üblicherweise gesintert, als Auflage für die kleinen Teile. Die Medien welche benötigt werden um die Behandlung vorzunehmen, durchströmen diese Siebplatte und erzeugen ein sogenanntes Wirbelbett, welches die Behandlung bewirkt. Nach erfolgter Behandlung werden die Teile wie bei der vorangehend beschriebenen Einrichtung in diesem Behälter zum Verbraucher transportiert und an diesen übergeben. Nachteilig bei diesem Verfahren ist, dass der Behandlungsbehälter rotiert werden muss, was verfahrenstechnisch sehr aufwendig ist. Sämtliche Anschlüsse müssen rotationsfähig ausgelegt sein und dazu muss die Abkoppelung unter sterilen Bedingungen erfolgen, damit eine Ankoppelung an den Verbraucher wieder unter sterilen Bedingungen erfolgen kann. Ein weiterer gravierender Nachteil ist, dass die Teile sofort "verbraucht", d.h. nach der Behandlung sofort an den Verbraucher übergeben werden müssen. Eine Zwischenlagerung unter sterilen Bedingungen ist zwar möglich, aber wenn es um grössere Mengen geht und eine Zwischenlagerung der Teile notwendig ist, müssen mehrere Behandlungsbehälter vorhanden sein. Dies bedeutet hohe Investitionen, da solche Behandlungsbehälter komplex sind und stark instrumentiert werden müssen, um die Behandlungsanforderungen abzudecken.

Es sind auch rotierende Behandlungsbehälter bekannt, die nicht als Transportbehälter verwendet werden, sondern fest installiert sind. Nach der Behandlung werden die Teile in meistens fahrbare Transportbehälter abgelassen und dort unter sterilen Bedingungen gelagert, bis sie an den Verbraucher übergeben werden können.

Auch bei solchen Einrichtungen besteht der Nachteil, dass der Behandlungsbehälter rotiert werden muss, was verfahrenstechnisch sehr aufwendig ist, da sämtliche Anschlüsse rotationsfähig ausgelegt sein müssen.

Aus WO03/009942 ist eine Vorrichtung zum Aufbereiten von Inkontinenzartikeln bekannt, die in einem äusseren Gehäuse eine um eine horizontale Achse drehbare perforierte Trommel aufweist. Die umlaufende Wand der Trommel ist mit Durchbrechungen versehen. Es sind Einfüllöffnungen zum Einfüllen von Inkontinenzartikel in die Trommel vorgesehen. Weiter sind am Gehäuse Zuläufe zur Zuführung von Waschwasser, etc. und ein Zugang für Zuluft sowie Pumpen zum Abführen von gelösten Bestandteilen und Absaugen von Luft vorgesehen. Das Waschwasser und die Zuluft verteilen sich sowohl im Innenraum der Trommel als auch im Inneren des Gehäuses, um gelöste Bestandteile aufzunehmen und anhaftende Bestandteile abzulösen.

Aus W02004/029351 ist eine Wäscheschleuder mit einer in einem Gehäuse mit hoher Drehzahl rotierenden perforierten Trommel bekannt. Separierte Flüssigkeit wird in Tanks abgeführt. Aus US 2,795,404 ist eine Mischvorrichtung mit einem im Inneren eines Gehäuses drehbaren kubischen Behälter bekannt, in welchem sich die zu mischenden Komponenten befinden. In keiner dieser bekannten Vorrichtungen kann ein Wirbelbett erzeugt werden, in welchem die schonende und gleichzeitig zuverlässige Behandlung von Kleinteilen möglich ist.

Aus DE 102 35 374 ist eine Vorrichtung zum Behandeln pharmazeutischer Behältnisse bekannt, bei welcher eine Gehäuseöffnung mit einem Fenster oder einer Tür verschlossen werden kann und mit einer aufblasbaren Dichtung versehen ist.

Der Erfindung liegt die Aufgabe zugrunde, die bestehenden Nachteile für die angegebenen Prozesse zu beheben.

Erfindungsgemäss wird dies erreicht durch einen stationären Behandlungsbehälter mit einem in dessen Innenraum angeordneten rotierenden Innenkorb nach Anspruch 1.

Der Erfindung liegen folgende Überlegungen zugrunde, die bei bekannten Einrichtungen nicht oder unzureichend berücksichtigt wurden und die somit in ihrer Gesamtheit neu sind. Für die Behandlung kleiner Teile sind grundsätzlich einige wichtige Prinzipien zu beachten:
- Die Teile erfordern schonende Behandlung. Dies kann erreicht werden durch sorgfältiges Drehen des umgebenden Behältnisses.
- Restwasser und Kondensat muss entleert werden. Die kleinen Teile enthalten oft Hohlräume, welche für eine gleichbleibende Behandlungsqualität entleert werden müssen. Dies kann ebenfalls durch sorgfältiges Drehen des umgebenden Behältnisses erreicht werden.
- Die Medien Dampf, Wasser, Luft müssen korrekt geführt werden, damit sie wirklich in Kontakt mit den zu behandelnden Teilen kommen und gewährleistet ist, dass alle Teile die qualitativ gleiche Behandlung erhalten.
- Da die Platzverhältnisse in den Anlagen meistens sehr beschränkt sind, ist es erforderlich Bauformen mit möglichst grossem Behandlungsvolumen und kleinen Aussenmassen zu entwickeln.
- Die Positioniergenauigkeit bei rotierenden Maschinen ist wichtig, damit die kleinen behandelten Teile den richtigen Materialfluss durchlaufen. Es ist z.B. sehr wichtig, dass keine Teile mehrfach behandelt werden, was passieren kann, wenn ein Teil nicht korrekt entladen wurde.

Im Folgenden werden anhand der beiliegenden Zeichnungen bevorzugte Ausführungsbeispiele der Erfindung beschrieben. Es zeigen
- Fig.1: eine schematische Darstellung der erfindungsgemässen Vorrichtung
- Fig.2: ein Detail der Verbindung zwischen Mantel und Innenkorb
- Fig.3: eine Version der Vorrichtung mit Kühleinrichtung
- Fig.4: eine schematisch dargestellte alternative Ausführungsform

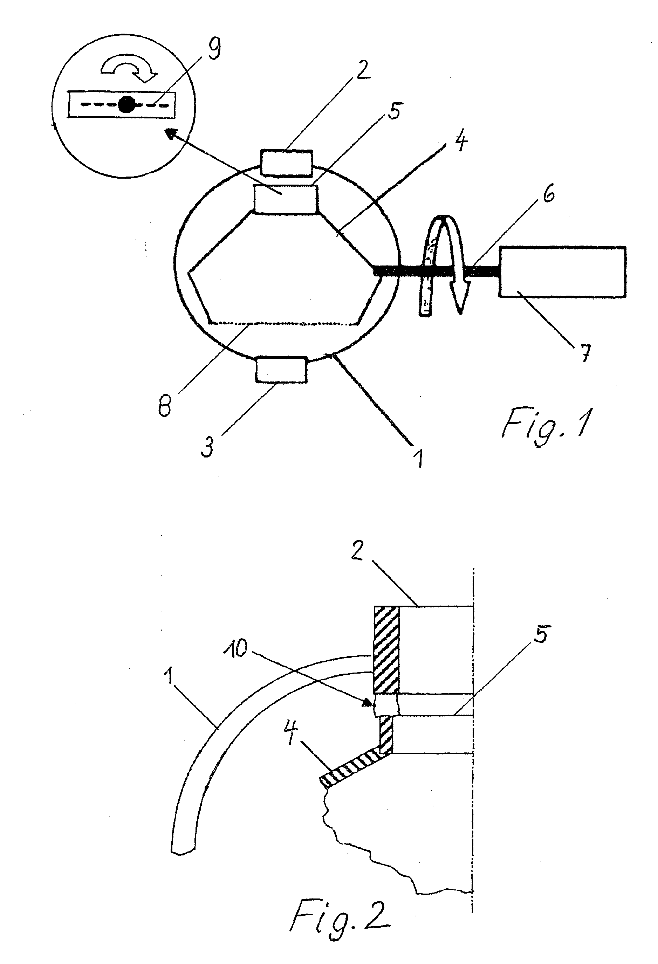

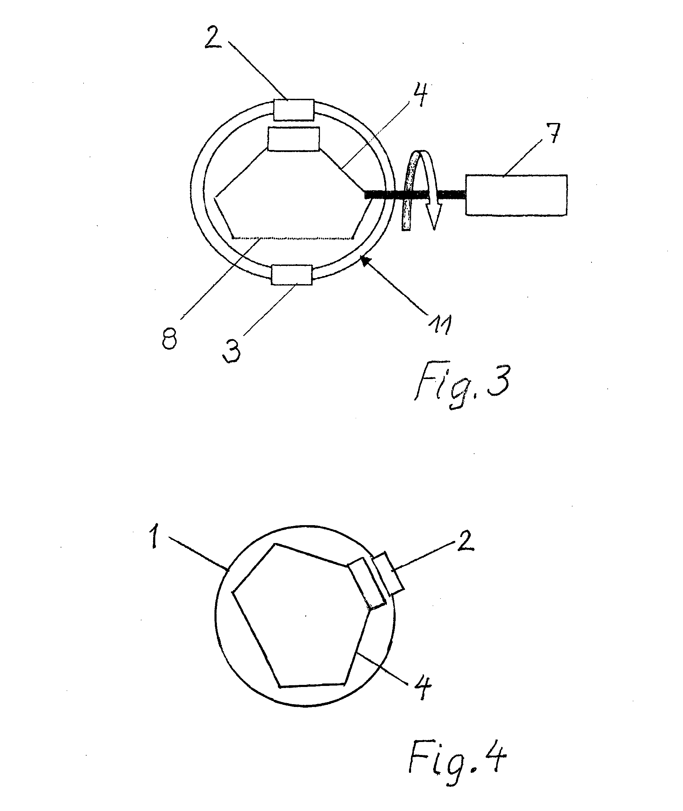

Der in Fig. 1 gezeigte kugelförmige Behandlungsbehälter 1 ist stationär, d.h. er ist fest installiert und hat keine Funktion als Transportbehälter. Damit sind eine einfache Installation und eine einfache Bauweise des Behandlungsbehälters möglich. An seiner Oberseite besitzt der Behälter eine Öffnung 2, durch welche die Beschickung erfolgt und an seiner Unterseite eine Entladeöffnung 3. Alternativ kann die Beschickungsöffnung auch, wie in Fig. 4 gezeigt, winkelversetzt seitlich angeordnet sein. Der Innenkorb wird dann für die Beschickung entsprechend gedreht. Der Behandlungsbehälter hat Kugelform, um das Totvolumen und das Verhältnis von Maschinengrösse zu Behandlungsvolumen möglichst klein zu halten. Diese Bauform ermöglicht es auch bei sehr engen Platzverhältnissen Behandlungsmaschinen mit grossem Behandlungsvolumen zu installieren.

Als Behältnis für die Behandlung der Teile ist im fest installierten Behandlungsbehälter 1 ein Innenkorb 4 vorgesehen, welcher über eine sich durch die Wand des Behandlungsbehälters erstreckende, waagrechte Welle 6 mit einem Antriebsmotor 7 verbunden ist. Aufgrund dieser Konstruktion wird der Innenkorb um eine horizontale Achse gedreht, was eine schonende Behandlung der Teile sichert. Der Innenkorb 4 ist mit einem Siebboden 8 aus einem feinmaschigen gesinterten Material ausgerüstet, welcher eine sehr feine Verteilung der Medien Luft, Wasser, Dampf bewirkt. Damit wird ein für die Behandlung perfektes Wirbelbett erzeugt, was eine homogene und gute Behandlungsqualität ergibt. Auf seiner dem Siebboden 8 gegenüberliegenden Seite ist der Innenkorb 4 mit einer Öffnung 5 versehen, die mit einer Siebklappe 9 ausgerüstet ist. Diese bewirkt, dass die Teile während der Behandlung im rotierenden Innenkorb verbleiben und die Entladung der Teile korrekt erfolgt.

Der Antriebsmotor 7 ist ein Schrittmotor. Dieser ermöglicht eine sehr genaue Positionierung insbesondere für die Positionen Beladen, Behandeln und Entleeren. Durch die präzise Positionierung wird vermieden, dass Teile an falsche Orte gelangen, z.B. nicht vollständig entladen werden.

Die Behandlungsmedien Luft, Wasser, Dampf etc. werden durch die unten am Behälter befindliche Entladeöffnung zu- und durch die oben angeordnete Öffnung 2 abgeführt. Um den Medienfluss korrekt zu steuern, ist, wie in Fig. 2 zu sehen, eine bewegliche Abdichtung 10 vorgesehen. Die Abdichtung verbindet den rotierenden Innenbehälter 4 mit dem Mantel 1 so, dass keine Medien aussen am Innenbehälter vorbei fliessen können, sondern alle notwendigen Medien durch den Siebboden 8 strömen und die Teile so durchströmen, dass eine einwandfreie Behandlung gewährleistet ist.

Die technische Realisierung wird mit einer aufblasbaren Dichtung 10 gelöst. Diese kann für den vorliegenden Anwendungsfall unter sterilen Bedingungen eingesetzt werden, da sie ähnlich einem in der Steriltechnik üblichen Membranventil funktioniert.

Mittels einfach zu bauenden Transportbehältern ist eine Zwischenlagerung der behandelten Teile unter sterilen Bedingungen möglich.

Wie in Fig. 3 gezeigt, kann der Behandlungsbehälter bei Bedarf mit einer Kühlung ausgestattet sein. Diese Kühlung wird mittels eines Doppelmantels 11 oder mittels einer Rohrschlange realisiert. Die Kühlung der Teile muss normalerweise unter sterilen Bedingungen erfolgen, das heisst, dass die Teile nach der Sterilisation mit Dampf nicht mehr mit flüssigen Medien in Berührung kommen dürfen. Die Kühlung der Teile erfolgt also mit einem Gas, z.B. Luft. Die Teile und auch der Behandlungsbehälter müssen gekühlt werden, was mit Gasen, welche eine sehr kleine Wärmekapazität haben, lange dauert und einen hohen Gasverbrauch erfordert. Eine Kühlung des Behandlungsbehälters mittels eines Kühlmediums, z.B. Kühlwasser ergibt kürzere Zykluszeiten für die Behandlung der Teile.

## Patentansprüche

1. Vorrichtung zum schonenden Reinigen, Sterilisieren und Trocknen grosser Mengen von kleinen Teilen in einem Wirbelbett mit einem stationären Behandlungsbehälter (1) mit einem in dessen Innenraum angeordneten, um eine horizontale Achse drehbaren Innenkorb (4) mit einem Siebboden, wobei der Innenkorb (4) auf seiner dem Siebboden (8) gegenüberliegenden Seite mit einer Öffnung (5) versehen ist, **dadurch gekennzeichnet, dass** eine bewegliche Abdichtung (10) zwischen Behandlungsbehälter (1) und Innenkorb (4) vorgesehen ist, wobei die Abdichtung (10) den rotierenden Innenkorb (4) mit dem Behandlungsbehälter (1) so verbindet, dass keine Medien aussen am Innenbehälter vorbei fliessen können.

2. Vorrichtung nach Anspruch 1, **dadurch gekennzeichnet, dass** die bewegliche Abdichtung (10) eine aufblasbare Dichtung ist.

3. Vorrichtung nach einem der Ansprüche 1 - 2, **dadurch gekennzeichnet, dass** der Behandlungsbehälter (1) Kugelform hat.

4. Vorrichtung nach einem der Ansprüche 1 - 3, **dadurch gekennzeichnet, dass** der Innenkorb mit einem ausserhalb des Behandlungsbehälters angeordneten Schrittmotor oder einem Servomotor (7) gedreht wird.

5. Vorrichtung nach einem der Ansprüche 1 - 4, **dadurch gekennzeichnet, dass** der Innenkorb mit einer dem Siebboden gegenüber angeordneten Siebklappe (9) versehen ist.

6. Vorrichtung nach einem der Ansprüche 1 - 5, **gekennzeichnet durch** eine am Behandlungsbehälter angeordnete Kühleinrichtung (11).

## Claims

1. A device for gently cleaning, sterilizing and drying large volumes of small parts in a fluid bed, comprising a stationary treatment container (1) having an inner basket (4) with a screen bottom, which inner basket is arranged in the interior of the treatment container is rotatable about a horizontal axis, wherein on its side opposite the screen bottom (8), the inner basket (4) is provided with an opening (5), **characterized in that** a movable seal (10) is provided between the treatment container (1) and the inner basket (4), wherein said seal (10) connects the rotating inner basket (4) to the treatment container (1) in such a manner that no media can flow past the outside of the inner container.

2. The device according to claim 1, **characterized in that** the movable seal (10) is an inflatable seal.

3. The device according to any one of the claims 1 to 2, **characterized in that** the treatment container (1) has a spherical shape.

4. The device according to any one of the claims 1 to 3, **characterized in that** the inner basket is rotated by a stepper motor or a servo motor that is arranged outside of the treatment container.

5. The device according to any one of the claims 1 to 4, **characterized in that** the inner basket is provided with a screen flap (9) that is arranged opposite to the screen bottom.

6. The device according to any one of the claims 1 to 5, **characterized by** a cooling unit (11) that is arranged on the treatment container.

## Revendications

1. Dispositif pour nettoyer, stériliser et sécher sans les abîmer de grandes quantités de petites pièces dans un lit fluidisé, comportant une cuve de traitement fixe (1), dotée d'un panier intérieur (4) agencé à l'intérieur de celle-ci, apte à tourner autour d'un axe horizontal, et ayant un fond de tamis, le panier intérieur (4) comportant une ouverture (5) sur son côté tourné à l'opposé du fond de tamis (8), **caractérisé en ce qu'**un joint d'étanchéité mobile (10) est disposé entre la cuve de traitement (1) et le panier intérieur (4), le joint d'étanchéité (10) reliant le panier intérieur tournant (4) avec la cuve de traitement (1) de telle sorte qu'il ne puisse pas s'écouler de milieux à l'extérieur de la cuve intérieure.

2. Dispositif selon la revendication 1, **caractérisé en ce que** le joint d'étanchéité mobile (10) est un joint gonflable.

3. Dispositif selon l'une des revendications 1 et 2, **caractérisé en ce que** la cuve de traitement (1) est de forme sphérique.

4. Dispositif selon l'une des revendications 1 à 3, **caractérisé en ce que** le panier intérieur est entraîné en rotation par un moteur pas-à-pas disposé à l'extérieur de la cuve de traitement ou par un servomoteur (7).

5. Dispositif selon l'une des revendications 1 à 3, **caractérisé en ce que** le panier intérieur est doté d'un clapet à tamis (9) disposé à l'opposé du fond de tamis.

6. Dispositif selon l'une des revendications 1 à 5, **caractérisé par** un dispositif de refroidissement (11) disposé sur la cuve de traitement.
